Europäisches Patentamt

European Patent Office

Office européen des brevets

(19)

(11) Publication number: **0 298 666 B1**

(12) **EUROPEAN PATENT SPECIFICATION**

(45) Date of publication of patent specification: **11.08.93**

(51) Int. Cl.⁵: **A61K 9/20**, A61K 31/19, A61K 47/00

(21) Application number: **88306003.0**

(22) Date of filing: **01.07.88**

(54) **Spray dried ibuprofen compositions.**

(30) Priority: **08.07.87 US 71116**

(43) Date of publication of application:
**11.01.89 Bulletin 89/02**

(45) Publication of the grant of the patent:
**11.08.93 Bulletin 93/32**

(84) Designated Contracting States:
**AT BE CH DE ES FR GB IT LI LU NL**

(56) References cited:
**EP-A- 0 040 472      EP-A- 0 070 970**
**EP-A- 0 130 683      EP-A- 0 137 668**
**EP-A- 0 159 631      EP-A- 0 172 014**
**EP-A- 0 206 291      FR-A- 2 496 461**

(73) Proprietor: **AMERICAN HOME PRODUCTS CORPORATION**
**685, Third Avenue**
**New York, New York 10017(US)**

(72) Inventor: **Ho, Ying Tien Richard**
**416 Avondale Avenue**
**Haddonfield, NJ 08033(US)**
Inventor: **Blank, Robert George**
**2966 Driftwood Lane**
**Vineland New Jersey(US)**

(74) Representative: **Brown, Keith John Symons et al**
**c/o Wyeth Laboratories Huntercombe Lane South**
**Taplow Maidenhead Berkshire SL6 0PH. (GB)**

Rank Xerox (UK) Business Services
(3.10/3.6/3.3.1)

**Description**

The invention relates to spray dried compositions comprising agglomerates of ibuprofen in a gelatinized starch matrix and to a method for manufacture thereof.

The commercial analgesic, aspirin, can be dry-mixed with starch and is then directly compressible into tablets. The commercial analgesic acetaminophen, on the other hand, cannot be similarly dry mixed and directly compressed but must be further processed such as, for example, by wet granulation; by spray drying acetaminophen with pre-gelatinized starch as described in European Pat. Appln. EP 40,472; or by fluidizing acetaminophen and cross-linked sodium carboxymethyl cellulose in hot air, pulverizing the mixture with pregelatinized starch paste, and drying as described in Fr. Demande FR 2,496,461. EP-A-0130683 discloses an acetaminophen composition capable of being directly tabletted which is prepared by spray drying acetaminophen, partially gelatinized starch and a lubricant.

The commercial analgesic ibuprofen is also different from aspirin in that it cannot be dry-mixed with starch and directly compressed into tablets. In the past, most ibuprofen tablets have been prepared from a wet granulation of a binder and ibuprofen which is dried, mixed with lubricants and disintegrating agents and compressed into tablets. This process involves several processing steps and is disadvantageous because of the equipment costs, time involved and higher rejection rate. A dry granulation process is described in United States Patent 4,609,675.

The present invention provides a spray dried ibuprofen composition which is directly compressible into tablets. The spray dried compositions are free flowing and simplify production of tablets to a simple mixing and tabletting operation with the necessity of only small amounts of conventional tabletting lubricants and with a minimal rejection rate of underweight or imperfect tablets. Moreover, tablets formed from the spray dried compositions have good physical stability in respect of friability, disintegration and dissolution properties, and have excellent bioavailability associated with high dosage levels of ibuprofen.

The spray dried ibuprofen compositions of this invention comprise finely divided ibuprofen in a gelatinized starch matrix and may be of a generally spherical shape. The spray dried compositions comprise ibuprofen, as a disintegrant crospovidone, croscarmellose sodium and/or sodium starch glycolate, pregelatinized starch, polyvinylpyrrolidone and/or sodium lauryl sulfate as a wetting agent and, optionally colloidal silica.

The proportions of ingredients in the spray dried ibuprofen compositions can be adjusted within limits to provide a free flowing, dust free powder suitable for feed to a high speed tablet press. The proportions generally range from about 80% to about 90% by weight ibuprofen, about 1.5% to about 6% by weight disintegrant, about 8% to about 12% by weight pregelatinized starch, about 0.10% to about 0.35% by weight colloidal silica, and about 0.2% to about 2.0% by weight wetting agent.

The spray dried compositions of the invention have several properties which make them especially suitable for charging to a high speed tablet press. One such property is the free flow characteristic as evidenced by a low angle of repose when collected, for example, in a beaker from a funnel positioned above the beaker. The free flow characteristic permits faster tablet compression with less weight variation between tablets. Another such property is the natural lubricity of the spray dried compositions, requiring only minimal amount of tabletting lubricants such as, for example, stearic acid. In general, the lower the lubricant level, the faster the dissolution of the tablet.

The ibuprofen is available commercially, for example, from Ethyl Corporation, Baton Rouge, Lousiana. A suitable pharmaceutical grade is marketed as a powder with a particle distribution of 100-250 microns (1-2.5 $\times\ 10^{-4}$ m).

Croscarmellose sodium is a cross-linked polymer of carboxymethylcellulose sodium and is available in Type A and Type B depending upon the degree of substitution. Type A is preferred for the compositions of this invention. It is sold under the trade name ACDISOL and is available from FMC Corporation, 200 Market Street, Philadelphia Pa. 19103, U.S.A.

Pregelatinized starch is starch that has been chemically and/or mechanically processed to rupture all or part of the granules separated from the mature grain of corn in the presence of water. It is available from National Starch & Chemical Corp., Bridgewater, N.J. U.S.A. as STARCH 1551.

The colloidal silica is a commercial product and is available commercially in USP and NF grades. It is marketed by Degussa Corp., Teterboro, N.J. U.S.A. and a suitable grade is marketed under the tradename AEROSIL 200.

Polyvinylpyrrolidone is available commercially in U.S.P. grade under the generic name povidone and is marketed by GAF Corporation, 140 West 51st. Street, New York, N.Y. 10020 under the trademark PLASDONE K.A suitable grade is PLASDONE K-29/32 which has a molecular weight of about 29,000-32,000. Crospovidone are cross linked insoluble homopolymers of polyvinylpyrrolidone also known as

2

EP 0 298 666 B1

polyvinylpolypyrrolidone. It is also available from GAF Corporation under the trademark POLYPLASDONE-XL-X.

Sodium lauryl sulfate is a commercial product in USP grade. Preferably it is used in powder form to promote blending and is available from Albright Wilsen, 180 Old Tappan Road, Old Tappan, N.J., U.S.A., under the tradename EMPICOL and a suitable grade is EMPICOL 0303. Sodium starch glycolate is available commercially and is marketed by Generichem Corp., 85 Main Street, Little Falls, N.J. under the trademark PRIMOJEL A. It is made from potato starch.

Spray dryers can be of the usual laboratory or commercial type. Suitable spray dryers are manufactured by Buchi Laboratoriums-Technik AG, by the Anhydro Company of Attleboro, Massachusetts and Niro Atomizer Inc., of Columbia, Maryland.

The spray dryer employed in the first six examples was a Niro Portable Spray Dryer, Model No. 21231-0001. The operating conditions include a variable air inlet temperature, a variable air pressure of compressed air driving the atomizer wheel, and a variable feed rate.

The invention will be further illustrated by the following examples in which the spray dried compositions were prepared using the procedure described therein. In the examples where indicated there were employed ibuprofen with a particle distribution of 100 to 250 microns ($1\text{-}2.5 \times 10^{-4}$ m), STARCH 1551 brand of pregelatinized starch, AEROSIL 200 brand of colloidal silica, ACDISOL Type A brand of croscarmellose sodium and PLASDONEK-29/32 brand of povidone, EMPICOL 0303 brand of sodium lauryl sulfate, and PRIMOJEL brand of sodium starch glycolate.

The invention will be further illustrated by the following examples in which the spray dried compositions were prepared using the procedures described therein.

## EXAMPLE 1

In this example the feed mixture charged to the spray dryer was composed of the following materials.

|  | Grams Ingredient | Weight % Ingredient in Suspension | Weight% Ingredient in Powder |
| --- | --- | --- | --- |
| Ibuprofen | 1000 | 35.9 | 85 |
| Pregelatinized Starch | 140 | 4.0 | 11.9 |
| Croscarmellose Sodium | 28 | .77 | 2.4 |
| Colloidal Silica | 3 | .08 | 0.26 |
| Povidone | 5 | .14 | 0.43 |
| Purified Water, deionized | 2414 | | |
| | 3590 | | |

The water was placed in a stainless steel mixing vessel equipped with a Lightnin mixer. The povidone, croscarmellose sodium, and colloidal silica were slowly added to the water with mixing and mixing was continued for 5 minutes. The ibuprofen powder was slowly dispersed in the mixture and mixed for 30 minutes until homogeneous. The pregellatinized starch was then slowly added and mixed until homogeneous.

The spray dryer was operated with a feed rate of 30 to 35 grams per minute and an air outlet temperature of 60° to 65°C. The atomizer pressure was 2.2 to 2.3 bar.

The product from the spray dryer was a fine powder with a moisture content of 1.17%.

3

## EXAMPLE 2

In this example the feed mixture charged to the spray dryer was composed of the following materials.

| | Grams Ingredient | Weight % Ingredient in Suspension | Weight% Ingredient in Powder |
|---|---|---|---|
| Ibuprofen | 500 | 23.94 | 85 |
| Pregelatinized Starch | 50 | 2.39 | 8.5 |
| Croscarmellose Sodium | 35 | 1.67 | 5.94 |
| Colloidal Silica | 1.5 | .07 | 0.25 |
| Povidone | 2 | .09 | 0.34 |
| Purified Water, deionized | 1500 | | 100% |
| | 2088.5 | | |

The water was placed in a stainless steel mixing vessel equipped with a Lightnin mixer. Slowly added to the water with mixing were the croscarmellose sodium, the colloidal silica and the povidone. Mixing was continued for 5 minutes. The ibuprofen was then slowly added to the mixture and mixing was continued for 30 minutes. The pregelatinized starch was then added and mixing continued for 20 minutes.

The spray dryer was operated with a feed rate of 20-25 grams per minute and the air inlet heater was set to produce an air outlet temperature of 60°-65°C. The atomizer pressure was 4 bar.

The product from the spray drier was a fine powder.

## EXAMPLE 3

In this example the feed mixture charged to the spray dryer was composed of the following materials.

| | Grams Ingredient | Weight% Ingredient in Powder |
|---|---|---|
| Ibuprofen | 500 | 85 |
| Pregelatinized Starch | 62.5 | 10.63 |
| Croscarmellose Sodium | 20.0 | 3.40 |
| Colloidal Silica | 1.5 | 0.17 |
| Povidone | 4.0 | 0.68 |
| Purified Water, deionized | 1512 | 100% |
| | 2100 | |

The water was placed in a stainless steel mixing vessel equipped with a Lightnin mixer. Slowly added to the water with mixing were the croscarmellose sodium, the colloidal silica and the povidone. Mixing was continued for 5 minutes. The ibuprofen was then slowly added to the mixture and mixing was continued for 30 minutes. The pregelatinized starch was then added and mixing continued for 2 hours.

The spray dryer was operated with a feed rate of 30 grams per minute and the air inlet heater was set to produce an air outlet temperature of 60°-65°C. The atomizer pressure was 80 p.s.i (about $5.5 \times 10^5$ Pa)

The product from the spray drier was a fine powder of about 40 microns ($4 \times 10^{-5}$ m) with a moisture content of 0.4% and a bulk density of 0.401 grams per cubic centimeter.

The above spray dried powder was blended into a formulation for a compressed tablet as follows:

|  | Grams Per Batch | Milligrams Per Tablet |
|---|---|---|
| Spray Dried Powder (85% ibuprofen) | 152.95 | 235.3 |
| Croscarmellose Sodium, Type A | 9.75 | 15.0 |
| Pregelatinized Starch | 19.50 | 30.0 |
| Compressible starch | 16.25 | 25.0 |
| Colloidal Silica | 0.13 | 0.2 |
| Sodium lauryl sulfate | 0.33 | 0.5 |
| Stearic acid | 1.30 | 2.0 |
|  |  | 308.0 |

The compression mix for tabletting was prepared by separately mixing the spray dried powder with the croscarmellose sodium, the pregellatinized starch and the compressible starch for 10 minutes in a PK-Blender and then mixing with a pre-screened [30 mesh Tyler; "mesh" refers to the number of divisions per inch (ie about 2.54 cm)] mixture of the colloidal silica, the sodium lauryl sulfate and the stearic acid powder.

The compression mix was tabletted on a Stokes Rotary Press using tablet tooling set up for double compression with a release pressure of 2.6 tons at a RPM of 14. The compression mix showed good flow characteristics.

The tablets were divided into two batches and the physical characteristics of the tablets are shown below:

|  | Batch 1 | Batch 2 |
|---|---|---|
| Average weight, mg/tab | 308-309 | 308-310 |
| Thickness, inch | 0.206-0.208 (about 0.523-0.528 cm) | 0.206-0.208 (about 0.523-0.528 cm) |
| Hardness, Strong Cobb Units | 6-8 | 4-6 |
| Disintegration Time, minutes | 1.25-1.50 | 1.25 |
| Friability, USP Test | 0-0.1 | 0-0.1 |

## EXAMPLE 4

In this example the feed mixture charged to the spray dryer was composed of the following materials.

|  | Grams Ingredient | Weight% Ingredient in Powder |
|---|---|---|
| Ibuprofen | 500 | 85 |
| Pregelatinized Starch | 62.5 | 10.63 |
| Sodium Starch Glycolate | 20.0 | 3.40 |
| Colloidal Silica | 1.5 | 0.26 |
| Povidone | 4.0 | 0.68 |
| Purified Water, deionized | 1512 |  |
|  | 2100 |  |

The water was placed in a stainless steel mixing vessel equipped with a Lightnin mixer. Slowly added to the water with mixing were the sodium starch glycolate, the colloidal silica and the povidone. Mixing was continued for 5 minutes. The ibuprofen was then slowly added to the mixture and mixing was continued for 30 minutes. The pregelatinized starch was then added and mixing continued for 1 hour.

The spray dryer was operated with a feed rate of 32 grams per minute and the air inlet heater was set to produce an air outlet temperature of 60°-65°C. The atomizer pressure was 80 p.s.i. (about $5.5 \times 10^5$ Pa).

The product from the spray drier was a fine powder of about 40 microns with a moisture content of 0.8% and a bulk density of 0.380 grams per cubic centimeter. The particle size distribution (Tyler screen) was, as percent retained on 100 mesh, 0.5%; retained on 200 mesh, 7.5%; retained on 325 mesh, 68.2% and greater than 325 mesh, 24.2%.

The above spray dried powder was blended into a formulation for a compressed tablet as follows:

|  | Milligrams Per Tablet |
| --- | --- |
| Spray Dried Powder (85% ibuprofen) | 257.4 |
| Sodium starch glycolate | 10.0 |
| Pregelatinized Starch | 20.0 |
| Compressible starch | 40.0 |
| Magnesium Stearate | 0.8 |
| Stearic acid | 2.0 |
|  | 330.0 |

The compression mix for tabletting was prepared by separately mixing the spray dried powder with the sodium starch glycolate, the pregellatinized starch and the compressible starch for 8 minutes in a PK-Blender and then mixing with a pre-screened (30 mesh Tyler) mixture of the magnesium stearate and the stearic acid powder.

The compression mix was tabletted on a Stokes Rotary Press using tablet tooling set up for double compression with a release pressure of 2.6 tons at a RPM of 14. The compression mix showed good flow characteristics.

The physical characteristics of the tablets are shown below:

|  | Batch |
| --- | --- |
| Average weight, mg/tab | 330 |
| Thickness, inch | 0.212-0.216 (about 0.538 - 0.548 cm) |
| Hardness, Strong Cobb Units | 4-6 |
| Friability, USP Test | 0-0.1 |

## EXAMPLE 5

In this example the feed mixture charged to the spray dryer was composed of the following materials.

|  | Grams Ingredient | Weight% Ingredient in Powder |
| --- | --- | --- |
| Ibuprofen | 500 | 84.96 |
| Pregelatinized Starch | 60 | 10.20 |
| Croscarmellose Sodium | 25 | 4.25 |
| Colloidal Silica | 1.5 | 0.25 |
| Sodium Lauryl Sulfate | 2.0 | 0.34 |
| Purified Water, deionized | 1500 | 100% |
|  | 20885 |  |

The water was placed in a stainless steel mixing vessel equipped with a Lightnin mixer. Slowly added to the water with mixing were the croscarmellose sodium, the colloidal silica and sodium lauryl sulfate. Mixing was continued for 5 minutes. The ibuprofen was then slowly added to the mixture and mixing was continued for 30 minutes. The pregelatinized starch was then added and mixing continued for 20 minutes.

The spray dryer was operated with a feed rate of 20-25 milliliters per minute and the air inlet heater was set to produce an air outlet temperature of 60°-63°C. The atomizer pressure was 3.5 bar.

The product from the spray drier was a fine powder.

The above spray dried powder was blended into a formulation for a compressed tablet as follows:

|  | Milligrams Per Tablet |
|---|---|
| Spray Dried Powder (85% ibuprofen) | 235.3 |
| Croscarmellose Sodium, Type A | 20.0 |
| Pregelatinized Starch | 60.0 |
| Colloidal Silica | 0.20 |
| Sodium lauryl sulfate | 0.50 |
| Stearic acid | 2.0 |
|  | 320.0 |

The compression mix for tabletting was prepared by separately mixing the spray dried powder with the croscarmellose sodium, the pregellatinized starch and the compressible starch for 10 minutes in a PK-Blender and then mixing with a pre-screened (30 mesh Tyler) mixture of the colloidal silica, the sodium lauryl sulfate and the stearic acid powder.

The compression mix was tabletted on a Stokes Rotary Press using tablet tooling set up for double compression with a release pressure of 2.6 tons at a RPM of 14. The compression mix showed good flow characteristics.

The physical characteristics of the tablets are shown below:

|  | Batch |
|---|---|
| Average weight, mg/tab | 320 |
| Thickness, inch | 0.212-0.215 (about 0.538 - 0.546 cm) |
| Hardness, Strong Cobb Units | 4-5 |
| Disintegration Time, minutes | 1-1.25 |
| Friability, USP Test | 0-0.1 |

## EXAMPLE 6

In this example the feed mixture charged to the spray dryer was composed of the following materials.

|  | Grams Ingredient | Weight% Ingredient in Powder |
|---|---|---|
| Ibuprofen | 500 | 85.03 |
| Pregelatinized Starch | 50 | 8.5 |
| Croscarmellose Sodium | 35 | 5.59 |
| Colloidal Silica | 1.5 | 0.26 |
| Sodium Lauryl Sulfate | 1.5 | 0.26 |
| Purified Water, deionized | 1400 | 100% |

The water was placed in a stainless steel mixing vessel equipped with a Lightnin mixer. Slowly added to the water with mixing were the croscarmellose sodium, the colloidal silica and the sodium lauryl sulfate. Mixing was continued for 5 minutes. The ibuprofen was then slowly added to the mixture and mixing was continued for 30 minutes. The pregelatinized starch was then added and mixing continued for 20 minutes.

The spray dryer was operated with a feed rate of 15-17 milliliters per minute and the air inlet heater was set to produce an air outlet temperature of 60°-65°C. The atomizer pressure was 3.5 bar.

The product from the spray drier was a fine powder.

The above spray dried powder was blended into a formulation for a compressed tablet as follows:

|  | Milligrams Per Tablet |
|---|---|
| Spray Dried Powder (85% ibuprofen) | 235.3 |
| Croscarmellose Sodium, Type A | 20.0 |
| Pregelatinized Starch | 30.0 |
| Compressible Starch | 20.00 |
| Colloidal Silica | 0.20 |
| Sodium lauryl sulfate | 0.50 |
| Stearic acid | 2.0 |
|  | 308.0 |

The compression mix for tabletting was prepared by separately mixing the spray dried powder with the croscarmellose sodium, the pregellatinized starch and the compressible starch for 10 minutes in a PK-blender and then mixing with a pre-screened (30 mesh Tyler) mixture of the colloidal silica, the sodium lauryl sulfate and the stearic acid powder.

The compression mix was tabletted on a Stokes Rotary Press using tablet tooling set up for double compression with a release pressure of 2.6 tons at a RPM of 14. The compression mix showed good flow characteristics.

**EXAMPLE 7**

In this example the feed mixture charged to the spray dryer was composed of the following materials.

|  | Grams Ingredient | Weight% Ingredient in Powder |
|---|---|---|
| Ibuprofen | 500.0 | 88.07 |
| Pregelatinized Starch | 50.0 | 8.81 |
| Crospovidone NF, XL | 10.0 | 1.76 |
| Colloidal Silica | 1.5 | 0.26 |
| Povidone | 6.25 | 1.10 |
| Purified Water, deionized | 1512 | 100% |
|  | 2100 |  |

The water was placed in a stainless steel mixing vessel equipped with a Lightnin mixer. Slowly added to the water with mixing were the crospovidone, the colloidal silica and the povidone. Mixing was continued for 5 minutes. The ibuprofen was then slowly added to the mixture and mixing was continued for 30 minutes. The pregelatinized starch was then added and mixing continued for 2 hours. The change to the spray drier contained 30% solids.

The spray dryer was operated with a feed rate of 30 grams per minute and the air inlet heater was set to produce an air outlet temperature of 60°-65°C. The atomizer pressure was 80 p.s.i. (about $5.5 \times 10^5$ Pa).

The product from the spray drier was a fine powder of about 40 microns with a moisture content of 0.4% and a bulk density of 0.401 grams per cubic centimeter.

The above spray dried powder was blended into a formulation for a compressed tablet as follows:

|  | Grams Per Batch | Milligrams Per Tablet |
|---|---|---|
| Spray Dried Powder (88% ibuprofen) | 113.6 | 227.2 |
| Croscarmellose Sodium, Type A | 9.0 | 18.0 |
| Pregelatinized Starch | 15.0 | 30.0 |
| Compressible starch | 15.0 | 30.0 |
| Colloidal Silica | 0.15 | 0.3 |
| Sodium lauryl sulfate | 0.30 | 0.6 |
| Stearic acid | 1.00 | 2.0 |
|  |  | 308.1 |

The compression mix for tabletting was prepared by separately mixing the spray dried powder with the croscarmellose sodium, the pregellatinized starch and the compressible starch for 10 minutes in a PK-blender and then mixing with a pre-screened (30 mesh Tyler) mixture of the colloidal silica, the sodium lauryl sulfate and the stearic acid powder.

The compression mix was tabletted on a Stokes Rotary Press using tablet tooling set up for double compression with a release pressure of 2.6 tons at a RPM of 14. The compression mix showed good flow characteristics.

The tablets were divided into two batches and the physical characteristics of the tablets are shown below:

| | Batch |
|---|---|
| Average weight, mg/tab | 308-309 |
| Thickness, inch | 0.206-0.209 |
| Hardness, Strong Cobb Units | 4-6 |
| Disintegration Time, minutes | 1-1.5 |
| Friability, USP Test, 4 min. | 0-0.1 |

## EXAMPLE 8

In this example, four large size runs were made with an eight foot diameter Bowen Pilot Unit spray dryer. The charges to the runs are shown in the following table in terms of weight percentage of each ingredient and solids content of the dispersion to the spray dryer.

Typically, the solids were added to the water in the Dryer Feed Slurry Make-up Tank and agitated for two hours. The final viscosity ranged between 175-200 cps (175-200 mPa.)s, the temperature between 24-26°C, and the pH about 5.4.

The spray dryer was operated at an air inlet temperature of 138°-143°C., an air outlet temperature of 60°-65°C, an atomizer RPM of 19,000-21,000 and a feed rate of about 120 pounds (about 54 kg) per hour. The yield was about 95%.

The spray dried powder from Run 1 typically had a loose bulk density of 0.372 grams per cubic centimeter, a moisture content of 1.0 per cent, and a sieve fraction % Alpine retained on + 200 mesh of 14.9, and retained on + 325 mesh of 49.0.

The spray dried powder from Run 2 typically had a loose bulk density of 0.354 grams per cubic centimeter, a moisture content of 0.86 per cent, and a sieve fraction % Alpine retained on + 200 mesh of 13.5 and retained on + 325 mesh of 42.0. The flow rate through the bulk densiometer was 43 seconds and the angle of repose was 39°.

The spray dried powder from Run 3 typically had a loose bulk density averaging 0.359 grams per cubic centimeter, a moisture content averaging 0.95 per cent, and a sieve fraction % Alpine retained on + 200 mesh averaging 12.1 and retained on + 325 mesh averaging 39.1.

The spray dried powder from Run 4 typically had a loose bulk density averaging 0.362 grams per cubic centimeter, a moisture content of 1.19 and a sieve fraction % Alpine retained on + 200 mesh of 4.1 and retained on +325 mesh of 25.1. The flow rate through the bulk densiometer was 42 seconds and the angle of repose was 39°.

| Ingredient | Run #1 Per Cent | Run #1 Weight Kilograms | Run #2 Per Cent | Run #2 Weight Pounds (kilograms) | Run #3 Percent | Run #3 Weight Kilograms | Run #4 Percent | Run #4 Weight Kilograms |
|---|---|---|---|---|---|---|---|---|
| Pregelatinized Starch | 10.00% | 9.091 | 10.47 | 20.94 (9.50) | 10.25 | 8.441 | 10.25 | 8.441 |
| Croscarmellose Sodium | 3.50% | 3.182 | 3.41 | 6.82 (3.09) | 3.50 | 2.882 | 3.50 | 2.882 |
| Colloidal Silica | 0.30% | 0.273 | 0.27 | 0.54 (0.24) | 0.25 | 0.206 | 0.25 | 0.206 |
| Povidone | 1.20% | 1.091 | 0.85 | 1.70 (0.77) | 1.00 | 0.824 | 1.00 | 0.824 |
| Ibuprofen | 85.00% | 77.274 | 85.00 | 170.00 (77.11) | 85.00 | 70.0 | 85.00 | 70.0 |
| Total | 100.00% | 90.91 | 100% | 200.00 | 100% | 82.553 | 100% | 82.353 |
| | | | | | | | | |
| Deionized, Filtered Water | 56 gallons (about 212 l) | | 56 gallons (about 212 l) | | 51 gallons (about 193 l) | | 51 gallons (about 193 l) | |
| PerCent Solids | 30% Solids | | 30% Solids | | 30% Solids | | 30% Solids | |

## EXAMPLE 9

In this example, a semi-production run was made on the Bowen Pilot Unit spray dryer used in the previous example. The dry ingredients in the charge to the spray dryer, as shown below, were dry blended

10

in a PK blender for 30 minutes.

| Ingredient | Per Cent | Weight Kilograms |
|---|---|---|
| Pregelatinized Starch | 10.25 | 41.0 |
| Croscarmellose Sodium | 3.50 | 14.0 |
| Colloidal Silica | 0.25 | 1.0 |
| Povidone | 1.00 | 4.0 |
| Ibuprofen | 85.00 | 340.0 |
| Total | 100.00% | 400.0 |
| Deionized, Filtered Water | 246 gallons (about 931 l) | |
| Per Cent Solids | 30% | |

The solids were added to the water in the Dryer Feed Slurry Make-up Tank and agitated for 55 hours.

After 42 hours 10 additional gallons (about 37.8 l) of water were added to the tank to maintain the solids content at about 30%. The final viscosity ranged between 500 to 750 cps (500 to 750 m Pa.s) the temperature between 22.2 and 32.3°C and the pH was 5.47.

The spray dryer was charged over a single day period from 1127 until 1647 hours. The spray dryer was operated at an air inlet temperature of 270°-275°F (about 132-135°C), an air outlet temperature of about 140°F (about 60°C), an atomizer RPM of 18,639 and a feed rate of about 0.77-0.82 gallons (about 2.91-3.10 l) per minute. The yield was about 95%.

Samples of product from the spray dryer were taken periodically and the loose bulk density, moisture content and particle size distribution are shown in the following table. The spray dried material was collected into 7 drums.

| Time | Moisture % | LBD g/cc | Sieve +200 | Fraction +325 | |
|------|------------|----------|------------|---------------|---|
| 1127 | .73 | .350 | 9.8 | 34.1 | |
| 1147 | 1.0 | .354 | 14.0 | 37.2 | Drum 1 |
| 1207 | 0.96 | .356 | 10.9 | 38.6 | |
| 1233 | 0.98 | .353 | 16.3 | 41.2 | |
| 1253 | | | 16.6 | 42.9 | |
| 1314 | .93 | .349 | 15.6 | 43.2 | Drum 2 |
| 1334 | | | | | |
| 1346 | .89 | .360 | 17.7 | 44.3 | |
| 1415 | .86 | .356 | 15.8 | 42.0 | |
| 1435 | .91 | .340 | 12.5 | 41.3 | Drum 3 |
| 1500 | | | | | |
| 1520 | .88 | .344 | 10.4 | 42.7 | Drum 4 |
| 1540 | | | 14.5 | 43.3 | |
| 1600 | .91 | .343 | 11.8 | 40.5 | |
| 1630 | .90 | .341 | 10.8 | 39.5 | Drum 5 |
| 1650 | .88 | .340 | 10.0 | 37.9 | |
| 1720 | 1.06 | .334 | 15.2 | 40.5 | Drum 6 |
| 1740 | | .350 | 20.6 | 46.0 | |
| 1810 | .93 | .349 | 17.1 | 48.4 | Drum 7 |
| 1830 | | | | | |

The flow rate, as measured in seconds required to empty a funnel having a top opening of four and one-quarter inches (about 10.79 cm) in diameter, a bottom opening of three-quarter inch (about 1.9 cm) in diameter and a height of 12 inches (about 30.48 cm), was determined and the angle of repose was determined by collecting the flow from the funnel in a 400 milliliter Nalgene beaker placed 4 inches (about 10.16 cm) below the funnel, the beaker having a top diameter of 3 7/8 inches (about 9.84 cm), a bottom diameter of 2 7/8 inches (about 7.30 cm) and a height of 4 3/8 inches (about 7.30 cm), measuring the distance from the top of the beaker to the top of the powder, and extrapolating from a conversion table. The results are shown below:

12

| Spray Dried Powder | | | |
|---|---|---|---|
| | Flow Rate, Seconds | Angle Of Repose ° | Loose Bulk Density (gm/cc) |
| Drum 1 | 56 | 37 | 0.397 |
| Drum 2 | 47 | 38 | 0.386 |
| Drum 3 | 48 | 37 | 0.401 |
| Drum 4 | 43 | 37 | 0.404 |
| Drum 5 | 65 | 39 | 0.395 |
| Drum 6 | 43 | 36 | 0.404 |
| Drum 7 | 35 | 42 | 0.395 |

For optimum tabletting results, the loose bulk density is in the range of about 0.35 to 0.38 grams per cubic centimeter, the moisture content is less than 1% and the particle distribution is such that sieve fraction % Alpine retained on + 200 mesh ranges from about 10 to about 15% and retained on + 325 ranges from about 40 to about 60%. The angle of repose is between about 20° to about 50°, preferably about 35° to about 45°.

## EXAMPLE 10

Tablets were made from the spray dried powders from Examples 8 and 9 each with the formulation below:

| Ingredient | Milligrams Per Tablet |
|---|---|
| Spray Dried Powder | 236.0 |
| Pregelatinized Starch | 22.5 |
| Compressible Starch | 22.5 |
| Croscarmellose Sodium, Type A | 18.0 |
| Colloidal Silica | 0.45 |
| Sodium lauryl sulfate | 0.75 |
| Stearic acid | 1.80 |
| Total | 302.0 |

The compression mix for tabletting was prepared by separately mixing the spray dried powder with the croscarmellose sodium, the pregelatinized starch and the compressible starch for 10 minutes in a PK-blender and then mixing with a pre-screened (30 mesh Tyler) mixture of the colloidal silica, the sodium lauryl sulfate and the stearic acid powder. The flow rate through the bulk densiometer, the bulk density and the angle of repose were measured and are shown in the following table.

| Compression Mix | | | |
|---|---|---|---|
| | Flow Rate Seconds | Loose Bulk Density (gm) | Angle of Repose ° |
| Run 1 | 45 | 0.394 | 36 |
| Run 2 | 38 | 0.398 | 27 |
| Run 3 | -- | --- | -- |
| Run 4 | -- | --- | -- |
| Drum 1 | 46 | 0.459 | 25 |
| Drum 2 | 52 | 0.476 | 18 |
| Drum 3 | 48 | 0.454 | 29 |
| Drum 4 | 33 | 0.423 | 27 |
| Drum 5 | 49 | 0.463 | 21 |
| Drum 6 | 42 | 0.453 | 25 |
| Drum 7 | 46 | 0.461 | 26 |

The compression mix was tabletted on a Stokes Rotary Press using tablet tooling set up for double compression with a release pressure of 2.6 tons at a RPM of 14. The compression mix showed good flow characteristics.

The physical characteristics of the tablets are shown below:

| | Example 8 | | | | Example 9 |
| --- | --- | --- | --- | --- | --- |
| | Run 1 | Run 2 | Run 3 | Run 4 | Drum 2 |
| Average weight, mg/tablet | 298 | 298 | 297 | 297 | 300 |
| Thickness, inch (about cm) | 0.209–0.212 (0.531–0.538 cm) | 0.210–0.212 (0.533–0.538 cm) | 0.207–0.210 (0.526–0.533 cm) | 0.208–0.211 (0.528–0.536 cm) | 0.206–0.211 (0.523–0.536 cm) |
| Hardness, Strong Cobb Units | 4.5–6.5 | 5.5–6.5 | 4.5–5.5 | 4.0–5.5 | 5.5 – 6.5 |
| Disintegration Time, minutes | 1.75–2 | 1.41–1.67 | 3–4 | 4.75–5.25 | 2 – 2.25 |
| Dissolution Rate, minutes* | 5 | 4 | 5.6 | 6 | 4 |
| Friability, USP | — | 0.17 | 1.176 | | — |
| Friability Stress | — | — | 0.5 | | 0.5 |

*Dissolution By FDA Method.
Ibuprofen Tablets, Capsules & Caplets
(Time for 80% to be dissolved)

14

## EXAMPLE 11

Tablets were made from the spray dried powders from Example 10, Drums 5, 3 and 4 respectively, from the following ingredients in the amounts listed.

| Ingredient | Run #1 Milligrams Per Tablet | Run #1 Weight Grams | Run #2 Milligrams Per Tablet | Run #2 Weight Grams | Run #3 Milligrams Per Tablet | Run #3 Weight Grams |
|---|---|---|---|---|---|---|
| Spray Dried Powder | 236.00 | 6726 | 236.00 | 7080 | 236.00 | 7080 |
| Pregellatinized Starch | 22.40 | 641.25 | 25.00 | 750 | 25.00 | 750 |
| Starch | — | — | 19.80 | 600 | 24.80 | 600 |
| Compressible Modified Starch | 22.40 | 641.25 | — | — | — | — |
| Croscarmellose Sodium, Type A | 18.00 | 513 | 18.00 | 540 | — | — |
| Crospovidone XL | — | — | — | — | 13.00 | 540 |
| Colloidal Silica | 0.65 | 14.25 | 0.65 | 13.5 | 0.65 | 13.5 |
| Sodium Lauryl Sulfate | 0.75 | 19.95 | 0.75 | 22.5 | 0.75 | 22.5 |
| Stearic Acid, TP | 1.80 | 51.3 | 1.80 | 54 | 1.80 | 54 |
| | 302.00 | | 302.00 | | 302.00 | |

The spray dried powder was screened through a #12 mesh screen and added to a PK blender, followed by the pregelatinized starch, the compressible starch, the croscarmellose sodium or crospovidone, and the mixture was blended for 8 minutes. The colloidal silica, sodium lauryl sulfate and the stearic acid were screened through a #30 mesh screen, added to the PK blender and blended for 9 minutes. The flow rate, the loose bulk density and the angle of response of the compression were measured and are shown below.

### Compression Mix

| | Flow Rate | Angle of Repose | Loose Bulk Density |
|---|---|---|---|
| Run 1 | 49 | 21 | 0.403 |
| Run 2 | 48 | 29 | 0.454 |
| Run 3 | 33 | 27 | 0.423 |

The compression mix was tabletted on a Stokes Rotary Press Model BB-2, a 35 station press with 10.5 millimeter (0.71 concavity) tooling at 14 RPM for one hour and 30 minutes. The compression mix showed good flow characteristics.

The physical characteristics of the tablets are shown below:

15

|  | Run 1 | Run 2 | Run 3 |
|---|---|---|---|
| Average weight, mg/tablet | 302-305 | 303 | 303 |
| Thickness, inch | 0.203-0.206 (0.516-0.523 cm) | 0.203-0.206 (0.516-0.523 cm) | 0.205-0.209 (0.521-0.531 cm) |
| Hardness, Strong Cobb Units | 5-8 | 5-7 | 6.5-7.5 |
| Disintegration Time, minutes | 2-2.25 | 4.16-4.67 | 2.27-2.40 |
| Dissolution Rate, minutes* | 5.5 | 5 | 6 |
| Friability, USP | 0 | 0 | 0 |
| Content Uniformity | 3.6% | 3.61 | 4.31 |
| Moisture Content, % | 1.31 | 1.38 | 1.88 |

*Dissolution By FDA Method Ibuprofen Tablets, Capsules (Time for 80% to be dissolved)

**EXAMPLE 12**

An 8 kilogram batch of the tablets from Example 11, Run 1 was sugar coated with a coating procedure described below in a Colton 20 inch (about 50.8 cm) coating pan rotating at 30 RPM. The various coating materials were poured by hand from a small container or sprayer over the rotating tablets (about 26,500).

A seal coat was applied in the amount of 159 ml from a small sprayer during a period of three minutes. The seal was Opaglos GS-2-0310, a shellac type seal coat marketed by Colorcon Inc. West Point, Pennsylvania. After spraying was complete, the tablets were permitted to tumble for 2 minutes and then the tablets were removed from the coating pan and placed in an oven-humidifier at 40° c for 8 hours. The seal coat was applied in the amount of 0.006 mg/tablet.

A sub coat was applied to 7.5 kilograms of the seal coated tablets (about 24,193). The sub coat contained the ingredients in the amounts listed below:

| Ingredient | Weight Percent | Weight Grams |
|---|---|---|
| Sucrose, NF | 67.3% | 4038 |
| Microcrystalline Cellulose, | 5.2% | 312 |
| Deionized Water | 27.5% | 1650 |
|  |  | 6000 |

The microcrystalline cellulose was added to the water in a mixing vessel equipped with a stirrer and mixed for 2 minutes at 40 RPM. The suspension was heated to 60°c and the sucrose was added while mixing. The suspension was maintained at 60° - 65° during the time the sub coat was applied in about 18 applications of 200 ml per application, each application being followed by a tumbling period of 10 minutes followed by a warm (30°-35°) air drying interlude of 5 minutes.

The average tablet weight from the over-humidifier is about 310 milligrams and the target weight for the tablet with the subcoat is 425 milligrams. Beginning with the fifteenth application, tablets were weighed to obtain an average tablet weight prior to the next application.

A coloring coat was next applied in about 14 applications at the rate of 60 ml. per application, each application being followed by a tumbling period of 4 minutes followed by a warm (30°-35°) air drying interlude of 2 minutes. The target average tablet weight is 450 milligrams. The coloring coat was a mixture of ingredients in the amounts shown below:

| Ingredient | Weight Percent | Weight Grams |
|---|---|---|
| Sucrose, NF | 63.2% | 448 |
| Coloring | 11.7% | 175 |
| Deionized Water | 25.1% | 376.5 |

The water was heated to 60°c and the sucrose was added with stirring and cooled to 40°c. Add the coloring agent to the solution and mix for 5 minutes. Maintain the solution at 45°c during the period of application to the tablets.

16

A polishing step was next employed in which a mixture of Opaglos GS-2-0310 and carnauba wax was applied to the tumbling tablets in an amount respectively of 0.028 and 0.1 milligram per tablet. Tumbling was continued for 30 minutes to complete the tablet coating procedure.

The coated tablets were tested for dissolution characteristeics in accordance with the method suggested by the FDA for ibuprofen tablets. This test method differs from the USP test method in that 80% of the ibuprofen is dissloved in 60 minutes at 50 RPM using a Type 2 paddle. The dissolution time was 12 minutes for the coated tablets of this example.

**Claims**

**Claims for the following Contracting States : AT, BE, CH, DE, FR, GB, IR, IT, LI, LU, NL**

1. A spray dried ibuprofen composition suitable for direct compression into tablets comprising a spray dried dispersion in water of ibuprofen, pregelatinized starch, a disintegrant selected from the class consisting of crospovidone, croscarmellose sodium and sodium starch glycolate and a wetting agent for the ibuprofen selected from the class consisting of polyvinylpyrrolidone and sodium lauryl sulfate.

2. A spray dried composition as claimed in claim 1 which additionally contains colloidal silica.

3. A spray dried composition as claimed in claim 1 comprising a spray dried dispersion in water of about 80% to about 90% by weight ibuprofen, about 1.5% to about 6% by weight of a disintegrant selected from the class consisting of crospovidone, croscarmellose sodium and sodium starch glycolate, about 8% to about 12% by weight pregelatinized starch, and about 0.2% to about 2% by weight of a wetting agent selected from the class consisting of polyvinylpyrrolidone and sodium lauryl sulfate.

4. A spray dried composition as claimed in claim 3 which additionally contains about 0.1% to about 0.35% by weight of colloidal silica.

5. A compression mix for compressed tablets containing ibuprofen which comprises the spray dried compression as claimed in any one of claims 1 to 4.

6. A compression mix as claimed in claim 5 which additionally contains starch, a disintegrant and a wetting agent.

7. A process for preparing a coated compressed tablet containing ibuprofen characterised in that a spray dried composition as claimed in any one of claims 1 to 4 is incorporated as the ibuprofen component.

8. A process as claimed in claim 7 in which the coated compressed tablet is a sugar coated compressed tablet.

**Claims for the following Contracting State : ES**

1. A process for preparing an ibuprofen composition suitable for direct compression into tablets which comprises spray drying a dispersion in water of ibuprofen, pregelatinized starch, a disintegrant selected from the class consisting of crospovidone, croscarmellose sodium and sodium starch glycolate and a wetting agent for the ibuprofen selected from the class consisting of polyvinylpyrrolidone and sodium lauryl sulfate.

2. A process as claimed in claim 1 in which the dispersion additionally contains colloidal silica.

3. A process as claimed in claim 1 in which the spray dried dispersion consists essentially of about 80% to about 90% by weight ibuprofen, about 1.5% to about 6% by weight of a disintegrant selected from the class consisting of crospovidone, croscarmellose sodium and sodium starch glycolate, about 8% to about 12% by weight pregelatinized starch and about 0.2% to about 2% by weight of a wetting agent selected from the class consisting of polyvinylpyrrolidone and sodium lauryl sulfate.

4. A process as claimed in claim 3 which additionally contains about 0.1% to about 0.35% by weight of colloidal silica.

**5.** A process for preparing a coated compressed tablet containing ibuprofen characterised in that the product of the process claimed in any one of claims 1 to 4 is incorporated as the ibuprofen component.

**6.** A process as claimed in claim 5 wherein the tablet is a sugar coated compressed tablet.

**Patentansprüche**
**Patentansprüche für folgende Vertragsstaaten : AT, BE, CH, DE, FR, GB, IT, LU, NL**

**1.** Sprühgetrocknete Ibuprofenzusammensetzung, die zum direkten Komprimieren zu Tabletten geeignet ist, umfassend eine sprühgetrocknete Dispersion von Ibuprofen, vorgelatinierter Stärke, einem Desintegriermittel ausgewählt aus der Klasse bestehend aus Crospovidon, Natriumcroscarmellose und Natriumstärkeglykolat und einem Netzmittel für das Ibuprofen ausgewählt aus der Klasse bestehend aus Polyvinylpyrrolidon und Natriumlaurylsulfat in Wasser.

**2.** Sprühgetrocknete Zusammensetzung nach Anspruch 1, die außerdem kolloidale Kieselerde enthält.

**3.** Sprühgetrocknete Zusammensetzung nach Anspruch 1, umfassend eine sprühgetrocknete Dispersion von etwa 80 bis etwa 90 Gew.% Ibuprofen, etwa 1,5 bis etwa 6 Gew.% eines Desintegriermittels ausgewählt aus der Klasse bestehend aus Crospovidon, Natriumcroscarmellose und Natriumstärkeglykolat, etwa 8 bis etwa 12 Gew.% vorgelatinierter Stärke und etwa 0,2 bis etwa 2 Gew.% eines Netzmittels ausgewählt aus der Klasse bestehend aus Polyvinylpyrrolidon und Natriumlaurylsulfat in Wasser.

**4.** Sprühgetrocknete Zusammensetzung nach Anspruch 3, die außerdem etwa 0,1 bis etwa 0,35 Gew.% kolloidale Kieselerde enthält.

**5.** Kompressionsgemisch für ibuprofenhaltige koprimierte Tabletten, das die sprühgetrocknete Zusammensetzung nach einem der Ansprüche 1 bis 4 umfaßt.

**6.** Kompressionsgemisch nach Anspruch 5, das außerdem Stärke, ein Desintegriermittel und ein Netzmittel enthält.

**7.** Verfahren zum Herstellen einer ibuprofenhaltigen, beschichteten, komprimierten Tablette, dadurch gekennzeichnet, daß eine sprühgetrocknete Zusammensetzung nach einem der Ansprüche 1 bis 4 als Ibuprofenkomponente eingearbeitet wird.

**8.** Verfahren nach Anspruch 7, in dem die beschichtete komprimierte Tablette eine zuckerbeschichtete komprimierte Tablette ist.

**Patentansprüche für folgenden Vertragsstaat : ES**

**1.** Verfahren zum Herstellen einer Ibuprofenzusammensetzung, die zum direkten Komprimieren zu Tabletten geeignet ist, das das Sprühtrocknen einer Dispersion von Ibuprofen, vorgelatinierter Stärke, einem Desintegriermittel ausgewählt aus der Klasse bestehend aus Crospovidon, Natriumcroscarmellose und Natriumstärkeglykolat und einem Netzmittel für das Ibuprofen ausgewählt aus der Klasse bestehend aus Polyvinylpyrrolidon und Natriumlaurylsulfat in Wasser umfaßt.

**2.** Verfahren nach Anspruch 1, in dem die Dispersion außerdem kolloidale Kieselerde enthält.

**3.** Verfahren nach Anspruch 1, in dem die sprühgetrocknete Dispersion im wesentlichen aus etwa 80 bis etwa 90 Gew.% Ibuprofen, etwa 1,5 bis etwa 6 Gew.% eines Desintegriermittels ausgewählt aus der Klasse bestehend aus Crospovidon, Natriumcroscarmellose und Natriumstärkeglykolat, etwa 8 bis etwa 12 Gew.% vorgelatinierter Stärke und etwa 0,2 bis etwa 2 Gew.% eines Netzmittels ausgewählt aus der Klasse bestehend aus Polyvinylpyrrolidon und Natriumlaurylsulfat besteht.

**4.** Verfahren nach Anspruch 3, wobei zusätzlich etwa 0,1 bis etwa 0,35 Gew.% kolloidale Kieselerde enthalten sind.

**5.** Verfahren zum Herstellen einer ibuprofenhaltigen, beschichteten, komprimierten Tablette, dadurch gekennzeichnet, daß das Produkt des Verfahrens nach einem der Ansprüche 1 bis 4 als Ibuprofenkomponente eingearbeitet wird.

**6.** Verfahren nach Anspruch 5, in dem die beschichtete komprimierte Tablette eine zuckerbeschichtete komprimierte Tablette ist.

**Revendications**
**Revendications pour les Etats contractants suivants : AT, BE, CH, DE, FR, GB, IT, LU, NL**

**1.** Composition d'ibuprofène séchée par atomisation se prêtant au pressage direct en comprimés, comprenant une dispersion séchée par atomisation dans de l'eau d'ibuprofène, d'amidon prégélatinisé, d'un désintégrant choisi dans la classe formée par la crospovidone, la croscarmellose sodique et l'amidonglycolate de sodium et d'un agent mouillant pour l'ibuprofène choisi dans la classe formée par la polyvinylpyrrolidone et le laurylsulfate de sodium.

**2.** Composition séchée par atomisation suivant la revendication 1, qui contient aussi de la silice colloïdale.

**3.** Composition séchée par atomisation suivant la revendication 1, qui comprend une dispersion, séchée par atomisation, dans de l'eau d'environ 80% à environ 90% en poids d'ibuprofène, d'environ 1,5% à environ 6% en poids d'un désintégrant choisi dans la classe formée par la crospovidone, la croscarmellose sodique et l'amidonglycolate de sodium, d'environ 8% à environ 12% en poids d'amidon prégélatinisé et d'environ 0,2% à environ 2% en poids d'un agent mouillant choisi dans la classe formée par la polyvinylpyrrolidone et le laurylsulfate de sodium.

**4.** Composition séchée par atomisation suivant la revendication 3, qui contient aussi environ 0,1% à environ 0,35% en poids de silice colloïdale.

**5.** Mélange pour pressage en comprimés pressés contenant de l'ibuprofène, qui comprend la composition séchée par atomisation suivant l'une quelconque des revendications 1 à 4.

**6.** Mélange pour pressage suivant la revendication 5, qui comprend aussi de l'amidon, un désintégrant et un agent mouillant.

**7.** Procédé de préparation d'un comprimé pressé enrobé contenant de l'ibuprofène, caractérisé en ce qu'une composition séchée par atomisation suivant l'une quelconque des revendications 1 à 4 est incorporée comme composant ibuprofène.

**8.** Procédé suivant la revendication 7, dans lequel le comprimé pressé enrobé est un comprimé pressé enrobé de sucre.

**Revendications pour l'Etat contractant suivant : ES**

**1.** Procédé de préparation d'une composition d'ibuprofène se prêtant au pressage direct en comprimés, qui comprend le séchage par atomisation d'une dispersion dans de l'eau d'ibuprofène, d'amidon prégélatinisé, d'un désintégrant choisi dans la classe formée par la crospovidone, la croscarmellose sodique et l'amidonglycolate de sodium et d'un agent mouillant pour l'ibuprofène choisi dans la classe formée par la polyvinylpyrrolidone et le laurylsulfate de sodium.

**2.** Procédé suivant la revendication 1, dans lequel la dispersion contient aussi de la silice colloïdale.

**3.** Procédé suivant la revendication 1, dans lequel la dispersion séchée par atomisation consiste essentiellement en environ 80% à environ 90% en poids d'ibuprofène, environ 1,5% à environ 6% en poids d'un désintégrant choisi dans la classe formée par la crospovidone, la croscarmellose sodique et l'amidonglycolate de sodium, environ 8% à environ 12% en poids d'amidon prégélatinisé et environ 0,2% à environ 2% en poids d'un agent mouillant choisi dans la classe formée par la polyvinylpyrrolidone et le laurylsulfate de sodium.

4. Procédé suivant la revendication 3, qui contient aussi environ 0,1% à environ 0,35% en poids de silice colloïdale.

5. Procédé de préparation d'un comprimé pressé enrobé contenant de l'ibuprofène, caractérisé en ce que le produit du procédé suivant l'une quelconque des revendications 1 à 4 est incorporé comme composant ibuprofène.

6. Procédé suivant la revendication 5, dans lequel le comprimé est un comprimé pressé enrobé de sucre.

20